# EUROPÄISCHE PATENTANMELDUNG

(11) **EP 0 586 922 A1**
(43) Veröffentlichungstag der Anmeldung: **16.03.1994**
(21) Anmeldenummer: 93112997.7
(22) Anmeldetag: 13.08.1993
(51) Int. Cl.: C12M 3/06, C12M 3/04

(54) **Kulturgefäss für Zellkulturen**

(30) Priorität: 02.09.1992 DE 4229334
(71) Anmelder: Heraeus Instruments GmbH, D-63450 Hanau (DE)
(72) Erfinder: Falkenberg, Frank W., Prof.Dr., D-58456 Witten (DE)
(74) Vertreter: Kühn, Hans-Christian

(57) **Zusammenfassung**

Es ist ein rollflaschenähnliches Kulturgefäß (1) für Zellkulturen bekannt, das, eine Versorgungs-Kammer bildend, ein Nährmedium aufnimmt, in dem mindestens eine, die Zellkulturen aufnehmende Produktions-Kammer angeordnet ist, die von dem Nährmedium durch eine Dialysemembran (15) getrennt ist und mit einer Gas-Zu- und Abführung in die Versorgungskammer. Um hiervon ausgehend ein Kulturgefäß für die Erzeugung von Zellkulturen mit hoher Zelldichte bereitzustellen, das preisgünstig herstellbar und einfach handhabbar ist und bei dem die Gefahr von Infektionen vermindert ist, wird vorgeschlagen, daß als Gas-Zu- und Abführung die bei der Zellkultivierung benötigten und entstehenden Gase durchlässige Gasaustauschmembran (8) vorgesehen ist, die mindestens einen Teil der Außenwand des Kulturgefäßes bildet und deren Gesamtfläche, Material und Dicke so gewählt sind, daß die Durchlässigkeit für Sauerstoff pro 10⁷ Zellen mindestens 0,01 mg/h beträgt. Weiterhin sind Verfahren zur Zellkultivierung bekannt, bei denen in ein Kulturgefäß ein Nährmedium, das eine allseitig geschlossene und die zu kultivierende Zellkultur (23) aufnehmende Produktions-Kammer (15) umspült, gefüllt, und das Kulturgefäß in eine kontinuierliche Rollbewegung vesetzt wird. Um hiervon ausgehend ein Verfahren anzugeben, das einfach durchführbar ist und mit dem innerhalb kurzer Zeit hohe Zelldichten bei gleichzeitig hoher Vitalität erzeugbar sind, wird die Produktions-Kammer derart mit der zu kultivierenden Zellkultur gefüllt, daß sie eine Luftblase (24) enthält, deren Volumen 10 % des Volumens der Produktionskammer einnimmt und der Rollbewegung eine Taumelbewegung überlagert, derart, daß die Luftblase in der Zellkultur bewegt wird.

## Beschreibung

Die Erfindung betrifft ein rollflaschenähnliches Kulturgefäß für Zellkulturen mit einer Zelldichte von mindestens 10⁷ Zellen pro Milliliter, das, eine Versorgungs-Kammer bildend, ein Nährmedium aufnimmt, in dem mindestens eine, die Zellkulturen aufnehmende und von dem Nährmedium umspülbare Produktions-Kammer angeordnet ist, die von dem Nährmedium durch eine Dialysemembran, durch die von der Versorgungs-Kammer Nährstoffe in die Produktions-Kammer und von dort Stoffwechselprodukte In die Versorgungs-Kammer transportiert werden, getrennt ist, und mit einer bakteriendichten Gas-Zuführung in die Versorgungs-Kammer. Weiterhin betrifft die Erfindung ein Verfahren zur Zellkultivierung, wobei in ein rollflaschenähnliches Kulturgefäß ein Nährmedium, das eine allseitig geschlossene und die zu kultivierende Zellkultur aufnehmende Produktions-Kammer umspült, gefüllt, und das Kulturgefäß in eine kontinuierliche Rollbewegung vesetzt wird.

Derartige Kulturgefäße können beispielsweise für die in vitro-Gewinnung monoklonaler Antikörper eingesetzt werden. Monoklonale Antikörper werden heutzutage für zahlreiche Anwendungszwecke in Diagnostik, Therapie und biologisch-medizinischer Forschung üblicherweise nach Methoden der Hybridomtechnologie produziert. Als Hybridomzellen werden immortalisierte Hybride aus Antikörper-produzierenden Zellen und Myelomzellen bezeichnet. Die von den Hybridomzellen produzierten Antikörper, die sich durch eine hohe Spezifität auszeichnen, werden als "monoklonale" Antikörper bezeichnet.

Zur in vitro-Gewinnnung der Antikörper werden diese Hybridomzellen in bestimmten flüssigen Medien kultiviert, deren Zusammensetzung möglichst genau der des Blutes entspricht. Unter anderem enthalten diese Medien Salze, Zucker, Vitamine, Aminosäuren und ein auf Natrium-Hydrogencarbonat (NaHCO₃) basierendes Puffersystem. Üblicherweise werden die Hypridomzellen In einer Brutschrankatmosphäre mit hoher Luftfeuchtigkeit und einem CO₂-Gehalt, der mit dem im Medium enthaltenen NaHCO₃ im Gleichgewicht steht, kultiviert.

Für viele Anwendungszwecke der biologisch-medizinischen Grundlagenforschung, wie auch in der klinischen Diagnostik, sind die so nach konventionellen stationären in vitro-Methoden in Form von Gewebekulturüberstand gewonnenen monoklonalen Antikörper sehr gut geeignet. Für eine Reihe von Anwendungen, für die man monoklonale Antikörper in sehr reiner und hochkonzentrierter Form benötigt, sind die so gewonnenen monoklonalen Antikörper erst nach aufwendiger Weiterbearbeitung geeignet. Bei der in vivo-Produktionsform (Aszites-Flüssigkeit) sind im Ausgangsprodukt die monoklonalen Antikörper bereits in sehr hohen Konzentrationen (bis zu 20 mg/ml) enthalten. Bei der Gewinnung von monoklonalen Antikörpern durch die üblichen stationären in vitro-Produktionsformen werden jedoch nur Konzentrationen von ca. 0,01 bis 0,10 mg/ml erzielt.

Um die Herstellung monoklonaler Antikörper in höherer Konzentration und höherer Reinheit auch in vitro zu ermöglichen, wurde eine Vorrichtung In Form eines rollflaschenähnlichen Kulturgefäßes und ein Verfahren vorgeschlagen, bei dem eine "Versorgungs-Kammer" mit Nährstoffen für die zu versorgenden Zellen und mehrere darin angeordnete "Produktions-Kammern" in denen das Zellwachstum stattfindet und in denen die monoklonalen Antikörper produziert werden, voneinander durch semipermeable Dialysemembranen getrennt sind. Durch die semipermeable Dialysemembran hindurch werden die Zellen von der "Versorgungs-Kammer" aus mit Nährstoffen versorgt, während Abbau- und Stoffwechselprodukte ebenfalls durch die Dialysemembran aus den "Produktions-Kammern" in die "Versorgungs-Kammer" abgeführt werden. Diese Vorrichtung ist unter dem Namen "Bochumer Glasmaus" bekannt geworden. Dieses Kulturgefäß für Zellkulturen ist beispielsweise in dem Manuskript zu dem Poster-Vortrag "The Glassmmouse; A Rollerbottle-like Apparatus for Culturing Hybridomas in Dialysis Bags" von T. Hengelage, F. Haardt und F. W. Falkenberg, ausgestellt auf der Fachtagung "1991 World Congress on Cell and Tissue Culture", Anaheim, Kalifornien, vom 16. bis 20. Juni 1991, beschrieben. Das bekannte Kulturgefäß für Zellkulturen besteht aus einem Glasrohr mit einem Außendurchmesser von 120 mm, von dem die Enden in Form von Flanschen nach außen umgebogen sind. Die Länge des Glasrohres inklusive der Flansche beträgt 320 mm. Die Stirnseiten des Glasrohres sind mit 15 mm dicken PMMA-Scheiben verschlossen. Eine der PMMA-Scheiben weist 5 Durchgangsbohrungen auf, wovon eine in der Längsachse des Gefäßes ausgebildet und mit einem Stopfen verschlossen ist, der wiederum 2 kleinere Öffnungen aufweist, die als Einlaß für ein CO₂-Luft-Gemisch in das Gefäß und zum Druckausgleich dienen. Hierzu ist durch eine der beiden Öffnungen ein Edelstahlrohr mit einem Innendurchmesser von 1 mm hindurchgeführt, das bis zur gegenüberliegenden Stirnseite des Glasrohres reicht und durch das dem Inneren des Gefäßes über ein Sterilfilter das CO₂-Luft-Gemisch zugeführt wird. Die restlichen 4 Bohrungen der PMMA-Scheibe, die die zentrale Bohrung umgeben, dienen zur Einführung von Dialyse-Schläuchen, die in das Kulturgefäß hineinragen und deren Wandungen jeweils von einer semipermeablen Dialysemembran gebildet wird. In diese Dialyse-Schläuche werden die zu kultivierenden Zellkultur-Ansätze gefüllt; sie dienen als Produktions-Kammern, während der Innenraum des Kulturgefäßes im übrigen als Versorgungs-Kammer für die Zellen dient und bis etwa 40% des Volumens mit Nährmedium gefüllt ist. Durch die semipermeable Dialysemembran hindurch werden die Zellen von der Versorgungskammer aus mit Nährstoffen versorgt, während Abbau- und Stoffwechselprodukte ebenfalls durch die Dialysemembran abgeführt werden. Um ein Rotieren des Kulturgefäßes um seine Längsachse zu ermöglichen, kann dieses mit einer dichten Drehdurchführung versehen sein, durch die die Zuleitung für das CO₂-Luft-Gemisch hindurchgeführt ist.

Diese Vorrichtung, bei der die in die Produktions-Kammer eingeschlossenen Zellen von der semipermeablen Dialysemembran umgeben sind, ermöglicht die Kultivierung von Hypridomzellen über längere Zeiträume und in hoher Dichte (mehr als 10⁷ Zellen/ml). Bei dem bekannten Kulturgefäß handelt es sich jedoch um eine relativ aufwendige und kompliziert zu handhabende Vorrichtung, deren Bau einen gewissen Aufwand erfordert, den nicht jede Laborwerkstatt erbringen kann. Bei dem bekannten Kulturgefäß erfolgt die Versorgung mit dem für den Stoffwechsel der Zellkultur und für die Einstellungen der physiologischen Bedingungen notwendigen Gasen durch Einleiten des die umgebende Atmosphäre bildenden Gasgemisches in die Versorgungs-Kammer, wobei sich der Sauerstoff im Nährstoffmedium physikalisch löst und von dort durch die Dialysemembran in die Produktions-Kammer transportiert wird. Der Transport des Sauerstoffs von der Versorgungs-Kammer durch die Dialysemembran in die Zellkultur-Kammer ist zwar nicht sehr effektiv, reicht jedoch bis zu Zelldichten von ca. 10⁷ Zellen/ml aus. Für höhere Zelldichten bedarf es einer Verbesserung der Sauerstoffversorgung. Da bei hoher Zelldichte der Sauerstoffbedarf der Zellen so groß ist, daß der Gehalt an Sauerstoff in der Zellkultur-Kammer in wenigen Minuten, der in der Versorgungs-Kammer in weniger als 1 Stunde verbraucht ist, ist es notwendig, Sauerstoff aus der Gasphase durch Eintrag in das Nährmedium der Versorgungs-Kammer nachzuliefern. Als Schwachpunkt des bekannten Kulturgefäßes hat sich auch erwiesen, daß es durch die kontinuierliche Begasung mit dem CO₂-Luft-Gemisch über die Drehdurchführung zu Infektionen der Zellkulturen kommen kann.

Der vorliegenden Erfindung liegt die Aufgabe zugrunde, ein Kulturgefäß für die Erzeugung von Zellkulturen mit hoher Zelldichte bereitzustellen, das preisgünstig herstellbar und einfach handhabbar ist und bei dem die Gefahr von Infektionen vermindert ist. Weiterhin liegt der Erfindung die Aufgabe zugrunde, ein Verfahren zur Zellkultivierung anzugeben, das einfach durchführbar ist und mit dem innerhalb kurzer Zeit hohe Zelldichten bei gleichzeitig hoher Vitalität erzeugbar sind.

Hinsichtlich des Kulturgefäßes wird diese Aufgabe ausgehend von dem beschriebenen Kulturgefäß erfindungsgemäß dadurch gelöst, daß als Gas-Zu- und Abführung eine für Flüssigkeiten und die Zellkulturen kontaminierende Keime undurchlässige und für die bei der Zellkultivierung benötigten und entstehenden Gase durchlässige Gasaustauschmembran vorgesehen ist, die mindestens einen Teil der Außenwand des Kulturgefäßes bildet und deren Gesamtfläche, Material und Dicke so gewählt sind, daß die Durchlässigkeit für Sauerstoff pro 10⁷ Zellen mindestens 0,01 mg/h beträgt. Dadurch, daß als Gas-Zu- und Abführung eine für Flüssigkeiten und die Zellkulturen kontaminierenden Keime undurchlässige und für die für die Zellatmung notwendigen Gase durchlässige Gasaustauschmembran vorgesehen ist, können Infektionen der Zellkulturen, die über die Gas-Zu- bzw. Abführung eingeschleppt werden, nahezu ausgeschlossen werden. Als die für die Zellatmung notwendigen Gase gelten In erster Linie Sauerstoff und das beim Verbrauch des Sauerstoffs durch die Zellkultur entstehende Kohlendioxid. Da die Gasaustauschmembran für Sauerstoff und für Kohlendioxid durchlässig ist, können durch die Einstellung der Konzentrationen dieser Gase in der Atmosphäre, die das Kulturgefäß umgibt, die Konzentrationen innerhalb des Kulturgefäßes, insbesondere innerhalb der Versorgungs-Kammer beeinflußt werden. Der Mindest-Sauerstoffbedarf zum Überleben der Zellkulturen hängt unter anderem von der Art der Zellen ab. Im allgemeinen geht man von einem Mindest-Sauerstoffbedarf von 0,01 mg/h für je 10⁷ Zellen in der Zellkultur aus. Die Gesamtfläche, das Material und die Dicke der Gasaustauschmembran sind so zu wählen, daß diese "Mindest-Sauerstoff-Versorgung" gewährleistet wird. Geeignete Membran-Geometrien und -Gasdurchlässigkeiten lassen sich anhand weniger Versuche ermitteln.

Dadurch, daß die Gas-Zu- und Abführung mindestens einen Teil der Außenwand des Kulturgefäßes bildet, sind Gas-Zuleitungen bzw. -Ableitungen in Form von Rohr- oder Schlauchleitungen nicht erforderlich. Das Kulturgefäß ist daher preisgünstig herstellbar und, beispielsweise als Rollflasche ausgebildet, leicht handhabbar.

Als Zellkulturen kommen beispielsweise Hybridomzellen, Tumorzellen oder transfizierte Tumorzellen in Frage.

Insbesondere im Hinblick auf eine möglichst hohe Zelldichte hat sich ein Kulturgefäß bewährt, bei dem die Durchlässigkeit der Gasaustauschmembran für Sauerstoff pro 10⁷ Zellen in der Zellkultur mindestens 0,05 mg/h beträgt.

Für die Ausbildung der Gasaustausch-Membran haben sich Materialien als geeignet erwiesen, die für Sauerstoff einen Durchlässigkeitskoeffizienten von mindestens 1 x 10¹⁹ m²/s x Pa, vorteilhafterweise mindestens
5 x 10¹⁹ m²/s x Pa, aufweisen. Als besonders gut geeignete Materialien zur Ausbildung der Gasaustauschmembran hat sich Silikon erwiesen. Um eine ausreichende Sauerstoffversorgung zu gewährleisten, werden möglichst dünne Gasaustauschmembranen bevorzugt, bewährt haben sich Membranen mit einer Dicke zwischen 0,1 mm und 1 mm. Eine Silikon-Membran ist besonders preisgünstig und durch Spritzgußverfahren in jeder beliebigen Form herstellbar. Im Handel sind Silikone in vielen Dicken, Formen und definierten Gasdurchlässigkeiten erhältlich. Sie weist eine hohe Zerreißfestigkeit und eine gute chemische Beständigkeit gegenüber den üblicherweise bei der Zellkultivierung verwendeten Medien auf und ist insofern auch besonders einfach handhabbar. Besonders vorteilhaft ist weiterhin die einfache Sterilisierbarkeit einer Gasaustauschmembran aus Silikon; insbesondere ist sie besonders wirksam und ohne wesentliche Formänderung im Autoklaven sterilisierbar. Sie ist daher auch mehrfach verwendbar. Bei gegebener Gaspermeabilität hängt die erforderliche Geometrie der Gasaustauschmembran von dem durch die Zellatmung verursachten Gasbedarf sowie von den Partialdrücken der an der Zellatmung beteiligten Gase ab, insbesondere von dem von außen auf sie wirkenden Sauerstoffpartialdruck. Bei einem Außendruck von 1 atm und einer Brutschrankatmosphäre mit einem Sauerstoffpartialdruck etwa entsprechend der Luft, haben sich für Zellkulturen von 35 ml und 10⁷ Zellen pro Milliliter Zellkulturansatz, Gasaustausch-Membranen mit einer Fläche von mindestens 5 cm² als geeignet erwiesen.

Es hat sich als vorteilhaft erwiesen, das Kulturgefäß mit einer Gasaustauschmembran zu versehen, deren Fläche mindestens 5 cm² beträgt. Gasaustauschmembranen, die mindestens eine Fläche von 5 cm² aufweisen, haben sich beispielsweise bei Kulturgefäßen bewährt, die ein Volumen von ca. 300 ml beeinhalten, wobei die Zellkultur ca. 35 ml bei einer Zelldichte von etwa 10⁷ Zellen/ml umfaßt.

Besonders bewährt hat sich ein Kulturgefäß, das in Form eines Hohlzylinders ausgebildet ist und dessen Stirnseiten beidseitig mit einer Gasaustauschmembran verschlossen sind. Dabei gewährleistet die Mantelfläche des Hohlzylinders die mechanische Stabilität des Kulturgefäßes während durch die Gasaustauschmembran an den beiden Stirnseiten eine gute Gas-Versorgung und -Entsorgung der Zellkultur erreicht wird.

Insbesondere im Hinblick auf eine hohe Zelldichte innerhalb der Zellkultur hat sich ein Kulturgefäß als vorteilhaft erwiesen, bei dem die Gasaustauschmembran gefaltet ausgebildet ist. Dadurch wird bei gegebener, von der Gasaustauschmembran überspannter Fläche, die Gesamtoberfläche der Gasaustauschmembran vergrößert, so daß der Gasaustausch, insbesondere die Versorung der Zellen mit Sauerstoff, verbessert wird. Beispielweise kann bei hohlzylindrischen Kulturgefäßen die Mantelfläche in Form eines Faltenbalgs ausgebildet sein. Derartige Faltenbälge sind einfach herzustellen.

Insbesondere wird eine Ausführungsform des Kulturgefäßes bevorzugt, bei der die gesamte Außenwand, abgesehen von die Außenwand mechanisch stabilisierenden Stützelementen, als Gasaustauschmembran ausgebildet ist. Mit einem derartigen Kulturgefäß, das einen sehr guten Gasaustausch gewährleistet, sind hohe Zelldichten innerhalb kurzer Zeit und bei hoher Vitalität erzielbar. Die Stützelemente stabilisieren die Form des Kulturgefäßes und die geometrische Anordnung seiner Teile zueinander. Sie können beispielsweise als metallisches oder aus einem stabilen Kunststoff bestehendes Gitter, dessen Maschen von der Gasaustauschmembran überspannt werden, oder auch als aus dem gleichen Material wie die Gasaustauschmembran bestehende Verdickungen der Außenwand ausgebildet sein. Dabei haben sich besonders solche rollflaschenähnlichen Kulturgefäße als vorteilhaft erwiesen, bei denen auch die Stirnseiten des Kulturgefäßes als Stützelemente für die Gasaustauschmembran dienen, wobei die Mantelfläche im wesentlichen als Gasaustauschmembran ausgebildet ist.

Hinsichtlich des Verfahrens wird die Aufgabe, ausgehend von dem eingangs beschriebenen Verfahren, erfindungsgemäß dadurch gelöst, daß die Produktions-Kammer derart mit der zu kultivierenden Zellkultur gefüllt wird, daß sie eine Luftblase enthält, deren Volumen mindestens 10% des Volumens der Produktions-Kammer einnimmt, und daß der Rollbewegung eine Taumelbewegung des Kulturgefäßes überlagert wird, derart, daß die Luftblase infolge des Auftriebs in der Zellkultur bewegt wird. Infolge der Taumelbewegung des Kulturgefäßes und ihres Auftriebes in der flüssigen Zellkultur führt die Luftblase innerhalb der Produktions-Kammer eine Hin- und Her-Bewegung aus. Diese Hin- und Her-Bewegung der Luftblase trägt wesentlich zu einer guten Durchmischung des Zellkulturansatzes und damit zu seiner gleichmäßigen Versorgung mit Nährstoffen, insbesondere auch zu einem gleichmäßigen Sauerstoffeintrag, und stetigem Abtransport von Stoffwechselprodukten bei. Die Taumelbewegung kann beispielsweise durch eine zyklische Verkippung der Längsachse des Kulturgefäßes in Art einer Schaukelbewegung realisiert werden, wobei der Angelpunkt für die Schaukelbewegung beispielsweise im Bereich zwischen den Stirnseiten des Kulturgefäßes vorgesehen sein kann. Dies kann beispielsweise dadurch realisiert werden, daß bei einem rollflaschenähnlichen Kulturgefäß die Stirnseiten mit über die Mantelfläche hinausragenden Exzenterscheiben versehen sind, die als im Querschnitt ovale Abrollfläche wirken, wobei die Hauptachsen der beiden Ovale gegeneinander verdreht sind.

Vorteilhafterweise wird die Periode der Taumelbewegung gerade so lang gewählt, daß der Luftblase in jedem Bewegungszyklus genügend Zeit bleibt, bis zum jeweils erhöhten Ende der Produktions-Kammer aufzusteigen.

Der Durchmischungseffekt durch die Luftblase ist besonders ausgeprägt bei Luftblasen mit einem Volumen zwischen 20% und 50% des Volumens der Produktions-Kammer.

Die Geschwindigkeit der Rollbewegung des Kulturgefäßes wird vorteilhafterweise einerseits so schnell gewählt, daß sich die Zellkulturen innerhalb der Produktions-Kammer nicht absetzen können und andererseits so langsam eingestellt, daß die Zellen trotz ihrer trägen Masse der Rollbewegung noch folgen können. Die hierfür einzustellende Geschwindigkeit hängt von der Absenkgeschwindigkeit der Zellkulturen, von deren Masse und vom Umfang der Produktions-Kammer ab. Als Bahngeschwindigkeit für einen Punkt auf dem Umfang der Produktions-Kammer haben sich Werte zwischen 20 mm/s und 50 mm/s bewährt.

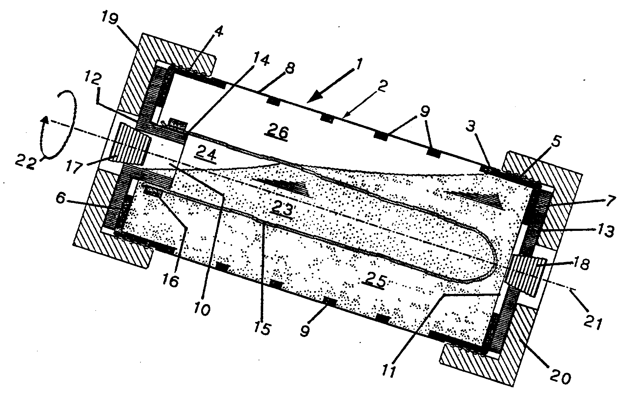

Ein Ausführungsbeispiel der Erfindung ist in der Zeichnung dargestellt und wird nachstehend näher erläutert. In der einzigen Figur der Patentzeichnung ist anhand einer schematischen Darstellung ein erfindungsgemäßes Kulturgefäß für Zellkulturen in einem Längsschnitt dargestellt.

Dem Kulturgefäß insgesamt ist die Bezugsziffer 1 zugeordnet. Es weist eine im wesentlichen hohlzylindrische Versorgungs-Kammer 2 auf, deren Außenwand von einem Silikonrohr 3 gebildet wird, das im Bereich der Stirnseiten mit Außengewinden 4;5 und mit nach Innen gerichteten Flanschen 6;7 versehen ist. Die Wandstärke des Silikonrohres 3 beträgt ca. 3 mm. Es weist über seine gesamte Mantelfläche gleichmäßig verteilte Durchbrüche auf, die von außen mit einer 0,38 mm dicken Silikonfolie 8 überspannt sind, so daß die in der Mantelfläche des Silikonrohres 3 verbleibenden, miteinander verbundenen Stege 9 von der Silikonfolie 8 aus in das Innere der Versorgungs-Kammer 2 hinneinragen und die, ein zusammenhängendes Gitter bildend, dem Kulturgefäß 1 eine für die bei der Kultivierung auftretenden Belastungen ausreichende mechanische Stabilität verleihen. Die Versorgungs-Kammer 2 hat eine Länge von ca. 15 cm, einen Außendurchmesser von ca. 5cm und vermag insgesamt ein Volumen von ca. 300 ml aufzunehmen. Die Mantelfläche des Silikonrohres 3 insgesamt beträgt etwa 240 cm², wovon etwa zwei Drittel die mit der Silikonfolie 8 überspannten Durchbrüche einnehmen. Die Stirnseiten des Silikonrohres 3 sind jeweils mit einer eine Mittenbohrung 10;11 aufweisenden Ringscheibe 12;13 aus stabilem Kunststoff verschlossen, wobei die Mittenbohrungen 10 der Ringscheibe 12 von einem hülsenförmigen Kragen 14 umgeben ist, der sich unter Ausbildung einer Art Schlaucholive von der Ringscheibe 12 in Richtung Innenraum der Versorgungs-Kammer 2 erstreckt. Über den Kragen 14 ist ein freies Ende eines Dialyseschlauches 15 (Visking 20/32) gezogen und mittels einer Silikonmanschette 16 daran befestigt. Das andere Ende des Dialyseschlauchs 15 ragt in die Versorgungs-Kammer 2 hinein und ist verschlossen. Der Dialyseschlauch 15, der aus einer dünnen, semipermeablen Dialysemembran (ebenfalls Bezugsziffer 15) besteht, vermag insgesamt ein Volumen von ca. 60 ml aufzunehmen. Die beiden Ringscheiben 12;13, deren Mittenbohrungen 10;11 jeweils mit einem Gummistöp-sel 17;18 verschließbar sind, werden mittels in die Außengewinde 4;5 der Versorgungs-Kammer 2 eingreifenden, ringförmigen Schraubdeckeln 19;20 flüssigkeitsdicht gegen die Flansche 6;7 der Versorgungs-Kammer 2 gepreßt. Die Gummistöpsel 17;18 sind über die Öffnungen der Schraubdeckel 19;20 zugänglich. Das Kulturgefäß 1 ist um seine Längsachse 21 rotierbar, wie dies mit dem Richtungspfeil 22 angedeutet ist.

In den Dialyseschlauch 15, der als Produktions-Kammer dient, werden ca 35 ml Zellkulturansatz 23 eingefüllt und die Produktions-Kammer anschließend mittels des Gummistöpsels 17 verschlossen. Dabei wird eine Luftblase 24 mit einem Volumen von ca. 25 ml in dem Dialyseschlauch 15 eingeschlossen. Gleichzeitig wird die Versorgungs-Kammer 2 bis etwa zu ihrer halben Höhe (bei waagerechter Orientierung) mit Nährmedium 25 für den Zellkulturansatz 23 gefüllt. Die Zellkultivierung wird In einem NaHCO₃-Puffer-abhängigen Medium vorgenommen. Zur Aufrechterhaltung des Puffersystems erfolgt die Kultivierung in einem in der Figur nicht dargestellten Brutschrank mit vorgegebener CO₂- und O₂-Atmosphäre, hoher Luftfeuchtigkeit und definierter Temperatur. Während der Rotation des Kulturgefäßes 1 wird der Dialyseschlauch 15 von dem Nährmedium 25 allseitig umspült. Dabei werden durch die semipermeable Dialysemembran 15 Nährstoffe von der Versorgungs-Kammer 2 in die Zellkultur (ebenfalls Bezugsziffer 23) transportiert und gleichzeitig von dort Stoffwechselprodukte in die Versorgungs-Kammer 2 abtransportiert. Der in der Brutschrank-Atmosphäre enthaltene Sauerstoff gelangt durch die Silikonfolie 8, deren Durchlässigkeit für Sauerstoff ausreichend ist, um den Sauerstoffbedarf der Zellkultur 23 von mindestens 0,01 mg/h pro 10⁷ Zellen zu decken, direkt sowohl in das Nährmedium 25, als auch in die darüber befindliche Versorgungskammer-Atmosphäre 26. Er wird im Nährmedium 25 physikalisch gelöst, durchdringt allmählich die Dialysemembran 15 und wird so in die Zellkultur 23 eingetragen. Das beim Verbrauch des Sauerstoffs entstehende Kohlendioxidgas wird durch die Dialysemembran 15 aus der Zellkultur 23 in das Nährmedium 25 der Versorgungs-Kammer 2 abgeführt und durch die Silikonfolie 8 aus dem Kulturgefäß 1 entfernt. Die Durchlässsigkeit der Silikonfolie 8 für Kohlendioxidgas ist wesentlich höher als diejenige für Sauerstoff, so daß innerhalb der Versorgungs-Kammer 2 insofern kein Überdruck entstehen kann. Für Flüssigkeiten und für die Zellkultur 23 eventuell kontaminierende Keime, wie Bakterien, Pilze oder Sporen ist die Silikonfolie 8 dagegen undurchlässig. Während der Kultivierung können über die jeweiligen Gummistöpsel 17;18 Proben entnommen, die Zellkultur 23 beimpft oder das Nährmedium 25 geprüft oder ausgetauscht werden.

Zum Zweck einer guten Durchmischung sowohl der Zellkultur 23 als auch des Nährmediums 25 kann das Kulturgefäß 1 mit einer Umdrehungsgeschwindigkeit von ca. 34 U/min um seine Längsachse 21 rotiert werden, wobei die Rotation 22 von einer langsamen, zyklischen Taumelbewegung des Kulturgefäßes 1 überlagert wird, bei der die Stirnseiten des Silikonrohres 3 relativ zueinander eine stetige Hin- und Herbewegung in Art einer Schaukelbewegung ausführen. Dabei bewegt sich infolge ihres Auftriebs in der Zellkultur 23 auch die Luftblase 24 innerhalb des Dialyseschlauchs 15 hin und her und unterstützt damit die Durchmischung der Zellkultur 23 und damit deren gleichmäßige Versorung mit Nährstoffen, insbesondere mit Sauerstoff, besonders wirkungsvoll.

Das erfindungsgemäße Kulturgefäß 1 ist besonders einfach handhabbar; mit ihm sind hochreine Zellkulturen mit Zelldichten von mehr als 10⁷ Zellen/ml, und, im Falle von Hybridomzellen, Konzentrationen von monoklonalen Antikörpern erreichbar, die mindestens 10fach höher als die mit normalen Standkulturen erzielbaren Konzentrationen liegen.

## Patentansprüche

1. Rollflaschenähnliches Kulturgefäß für Zellkulturen mit einer Zelldichte von mindestens 10⁷ Zellen pro Milliliter, das, eine Versorgungs-Kammer bildend, ein Nährmedium aufnimmt, in dem mindestens eine, die Zellkulturen aufnehmende Produktions-Kammer angeordnet ist, die von dem Nährmedium durch eine Dialysemembran, durch die von der Versorgungs-Kammer Nährstoffe in die Produktions-Kammer und von dort Stoffwechselprodukte in die Versorgungs-Kammer transportiert werden, getrennt ist, und mit einer Gas-Zu- und Abführung in die Versorgungs-Kammer, dadurch gekennzeichnet, daß als Gas-Zu- und Abführung eine für Flüssigkeiten und die Zellkulturen kontaminierende Keime undurchlässige und für die bei der Zellkultivierung benötigten und entstehenden Gase durchlässige Gasaustauschmembran (8) vorgesehen ist, die mindestens einen Teil der Außenwand des Kulturgefäßes (1) bildet und deren Gesamtfläche, Material und Dicke so gewählt sind, daß die Durchlässigkeit für Sauerstoff pro 10⁷ Zellen mindestens 0,01 mg/h beträgt.

2. Kulturgefäß nach Anspruch 1, dadurch gekennzeichnet, daß die Durchlässigkeit der Gasaustauschmembran (8) für Sauerstoff pro 10⁷ Zellen in der Zellkultur (23) mindestens 0,05 mg/h beträgt.

3. Kulturgefäß nach Anspruch 1 oder 2, dadurch gekennzeichnet, daß die Gasaustauschmembran (8) aus Silikon besteht, wobei die Gasaustauschmembran (8) eine Dicke zwischen 0,1 mm und 1 mm aufweist.

4. Kulturgefäß nach einem oder mehreren der Ansprüche 1 bis 3, dadurch gekennzeichnet, daß die Gasaustauschmembran (8) eine Gesamtfläche von mindestens 5 cm² überspannt.

5. Kulturgefäß nach einem oder mehreren der Ansprüche 1 bis 4, dadurch gekennzeichnet, daß das Kulturgefäß (1) in Form eines Hohlzylinders (3) ausgebildet ist, der an den Stirnseiten beidseitig mit der Gasaustauschmembran (8) verschlossen ist.

6. Kulturgefäß nach einem oder mehreren der Ansprüche 1 bis 5, dadurch gekennzeichnet, daß die Gasaustauschmembran (8) gefaltet ausgebildet ist.

7. Kulturgefäß nach einem oder mehreren der Ansprüche 1 bis 6, dadurch gekennzeichnet, daß die gesamte Außenwand (3) des Kulturgefäßes (1), abgesehen von die Außenwand (3) mechanisch stabilisierenden Stützelementen (9), als Gasaustauschmembran (8) ausgebildet ist.

8. Verfahren zur Zellkultivierung, wobei in ein rollflaschenähnliches Kulturgefäß nach einem oder mehreren der Ansprüche 1 bis 7 ein Nährmedium, das eine allseitig geschlossene und die zu kultivierende Zellkultur aufnehmende Produktions-Kammer umspült, gefüllt, und das Kulturgefäß in eine kontinuierliche Rollbewegung vesetzt wird, dadurch gekennzeichnet, daß die Produktions-Kammer (15) derart mit der zu kultivierenden Zellkultur (23) gefüllt wird, daß sie eine Luftblase (24) enthält, deren Volumen mindestens 10% des Volumens der Produktions-Kammer (15) einnimmt, und daß der Rollbewegung eine Taumelbewegung des Kulturgefäßes (1) überlagert wird, derart, daß die Luftblase (24) infolge des Auftriebs in der Zellkultur (23) bewegt wird.
